# EUROPEAN PATENT APPLICATION

(11) **EP 4 001 401 A1**
(43) Date of publication of application: **25.05.2022**
(21) Application number: 20836508.0
(22) Date of filing: 30.06.2020
(51) Int. Cl.: C12N 5/079, C12N 5/10, C12Q 1/06

(54) **PRODUCTION METHOD FOR CEREBRAL ORGANOID**

(30) Priority: 05.07.2019 JP 2019126266
(71) Applicant: Keio University, Tokyo, 108-8345 (JP)
(72) Inventor: ISHII, Hiroko, Tokyo 160-8582 (JP); OKANO, Hideyuki, Tokyo 160-8582 (JP)
(74) Representative: Cabinet Becker et Associés
(86) International application number: PCT/JP2020/025605
(87) International publication number: WO 2021/006107

(57) **Abstract**

A production method for a cerebral organoid having amyloid plaques is provided, the method including a step (a) of forming, in the presence of a SMAD inhibitor, an embryoid body from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a glycogen synthase kinase 3β (GSK3β) inhibitor to form an organoid; and a step (c) of removing the organoid after the step (b) from the extracellular matrix and subjecting the removed organoid to stirring culture in a medium, where at least a part of the step (c) is carried out in the presence of leukemia inhibitory factor (LIF).

## Description

### Technical Field

The present invention relates to a production method for a cerebral organoid. More specifically, the present invention relates to a production method for a cerebral organoid having amyloid plaques, a cerebral organoid having amyloid plaques, and a screening method for a prophylactic drug or therapeutic drug for Alzheimer's disease. Priority is claimed on Japanese Patent Application No. 2019-126266, filed on July 5, 2019, the content of which is incorporated herein by reference.

### Background Art

An organoid is a small organ that is formed by the accumulation of cells and has a structure and function similar to those of an organ in a living body. In recent years, studies on preparing various organoids from a pluripotent stem cell have been actively carried out, and for example, cerebral organoids, intestinal organoids, liver organoids, kidney organoids, and the like have been prepared.

Alzheimer's disease is an irreversible progressive cerebral disease. A characteristic structure called amyloid plaque is observed in the brain of Alzheimer's disease patients, and it is known that the constitutional component of the amyloid plaque is a peptide called amyloid β peptide. Amyloid β peptide is a peptide consisting of 36 to 43 amino acids in length, which is generated by cleavage of a precursor protein called β-amyloid precursor protein (APP).

An Alzheimer's disease model mouse has been developed for the development of therapeutic drugs for Alzheimer's disease. However, the structures of human and mouse brains are different from each other, and the findings obtained in the Alzheimer's disease model mouse may not be applicable to humans. As a result, there is a need for a human cerebral organoid that can more faithfully reproduce the pathophysiology of Alzheimer's disease and can be used in vitro. For example, Non-Patent Document 1 describes that a cerebral organoid having amyloid plaques has been prepared from an iPS cell derived from an Alzheimer's disease patient.

### Citation List

### Non-Patent Document

[Non-Patent Document 1]
Gonzalez C., et al., Modeling amyloid beta and tau pathology in human cerebral organoids., Mol Psychiatry, 23 (12), 2363-2374, 2018.

### Summary of Invention

### Technical Problem

However, there is room for improvement in the efficiency of forming amyloid plaques in the preparation method for cerebral organoids described in Non-Patent Document 1. An object of the present invention is to provide a technique for efficiently forming a cerebral organoid having amyloid plaques.

### Solution to Problem

The present invention includes the following aspects.
[1] A production method for a cerebral organoid having amyloid plaques, the method including a step (a) of forming, in the presence of a SMAD inhibitor, an embryoid body (EB) from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a glycogen synthase kinase 3β (GSK3β) inhibitor to form an organoid; and a step (c) of removing the organoid after the step (b) from the extracellular matrix and subjecting the removed organoid to stirring culture in a medium, in which at least a part of the step (c) is carried out in the presence of leukemia inhibitory factor (LIF).
[2] The production method according to [1], in which at least a part of the step (c) is carried out in the presence of more than 20% by volume of oxygen.
[3] The production method according to [1] or [2], in which the step (c) is carried out for 100 days or more.
[4] The production method according to any one of [1] to [3], further including a step of culturing, before the step (a), the pluripotent stem cell in the presence of less than 100 ng/mL of fibroblast growth factor-2 (FGF2).
[5] The production method according to any one of [1] to [4], in which the pluripotent stem cell is cultured in a feeder-free manner.
[6] The production method according to any one of [1] to [5], in which the Alzheimer's disease-related gene is a presenilin 1 (PS1) gene, a presenilin 2 (PS2) gene, or a β-amyloid precursor protein (APP) gene.
[7] A cerebral organoid having amyloid plaques of which the diameters are more than 20 µm.
[8] The cerebral organoid according to [7], in which in a cross section of the cerebral organoid, an average of a proportion of an area per amyloid plaque with respect to a total area of the cross section is 0.1% or more.
[9] The cerebral organoid according to [7] or [8], in which the number of the amyloid plaques is 2 or more per cerebral organoid.
[10] The cerebral organoid according to any one of [7] to [9], in which a molar ratio of an expression amount of amyloid β42 to an expression amount of amyloid β40 (the expression amount of amyloid β42/the expression amount of amyloid β40) is 0.15 or more.
[11] The cerebral organoid according to any one of [7] to [10], in which the cerebral organoid is produced by the production method according to any one of [1] to [6].
[12] A screening method for a therapeutic drug for Alzheimer's disease, the screening method including a step of culturing the cerebral organoid according to any one of [7] to [10] in the presence of a test substance; and a step of measuring the sizes of amyloid plaques of the cerebral organoid, in which a reduction of the sizes of the amyloid plaques indicates that the test substance is a therapeutic drug for Alzheimer's disease.
[13] A screening method for a prophylactic drug for Alzheimer's disease, the method including a step (a) of forming, in the presence of a SMAD inhibitor, an embryoid body from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a GSK3β inhibitor to form an organoid; a step (c) of removing the organoid after the step (b) from the extracellular matrix and subjecting the removed organoid to stirring culture in a medium to obtain a cerebral organoid, at least a part of the step (c) being carried out in the presence of a test substance; and a step (d) of measuring the sizes of amyloid plaques of the cerebral organoid, after carrying out the step (c) for 100 days or more, in which in the step (d), a reduction of the sizes of the amyloid plaques as compared with a control indicates that the test substance is a prophylactic drug for Alzheimer's disease.
[14] A screening method for a prophylactic drug or a therapeutic drug for Alzheimer's disease, the method including a step (a) of forming, in the presence of a SMAD inhibitor, an embryoid body from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a GSK3β inhibitor to form an organoid; a step (c) of removing the organoid after the step (b) from the extracellular matrix and subjecting the removed organoid to stirring culture in a medium to obtain a cerebral organoid, at least a part of the step (c) being carried out in the presence of a test substance; and a step (d') of quantifying expression amounts of amyloid β40 and amyloid β42 expressed by the cerebral organoid of the step (c), in which in the step (d'), a reduction of a ratio of an expression amount of amyloid β42 to an expression amount of amyloid β40 as compared with a control indicates that the test substance is a prophylactic drug or therapeutic drug for Alzheimer's disease.

### Advantageous Effects of Invention

According to the present invention, it is possible to provide a technique for efficiently forming a cerebral organoid having amyloid plaques.

### Brief Description of Drawings

Fig. 1 is a diagram showing a schedule for preparing cerebral organoids in Experimental Example 1.
Fig. 2A is a photomicrograph showing results of detecting amyloid plaques in human cerebral organoids in Experimental Example 2.
Fig. 2B is a photomicrograph showing results of detecting amyloid plaques in human cerebral organoids in Experimental Example 2.
Fig. 3 is a graph showing the proportion (%) of the area per amyloid plaque having a diameter of 20 µmor more with respect to the total area of the cross section of the human cerebral organoid in Experimental Example 2.
Fig. 4A is a photomicrograph showing results of detecting amyloid plaques in human cerebral tissues and human cerebral organoids in Experimental Example 3.
Fig. 4B is a photomicrograph showing results of detecting amyloid plaques in human cerebral tissues and human cerebral organoids in Experimental Example 3.
Fig. 4C is a photomicrograph showing results of detecting amyloid plaques in human cerebral tissues and human cerebral organoids in Experimental Example 3.
Fig. 4D is a photomicrograph showing results of detecting amyloid plaques in human cerebral tissues and human cerebral organoids in Experimental Example 3.
Fig. 4E is a photomicrograph showing results of detecting amyloid plaques in human cerebral tissues and human cerebral organoids in Experimental Example 3.
Fig. 5A is a graph showing results of quantifying the expression amount of amyloid β40 (Aβ40) expressed by cerebral organoids in Experimental Example 4.
Fig. 5B is a graph showing results of quantifying the expression amount of amyloid β42 (Aβ42) expressed by cerebral organoids in Experimental Example 4.
Fig. 5C is a graph showing results of calculating the molar ratio of an expression amount of amyloid β42 to an expression amount of amyloid β40 (the expression amount of amyloid β42/the expression amount of amyloid β40) of the cerebral organoid based on the results of Fig. 5A and Fig. 5B.
Fig. 6A is photographic images of cerebral organoids induced to differentiate using an iPS cell cultured in the presence of 100 ng/mL FGF2 in Experimental Example 5.
Fig. 6B is photographic images of cerebral organoids induced to differentiate using an iPS cell cultured in the presence of 10 ng/mL FGF2 in Experimental Example 5.
Fig. 7A is a fluorescence photomicrograph showing results of immunostaining of tau protein and βIII tubulin in cerebral organoids in Experimental Example 6.
Fig. 7B is a fluorescence photomicrograph showing results of immunostaining of tau protein and βIII tubulin in cerebral organoids in Experimental Example 6.
Fig. 7C is a fluorescence photomicrograph showing results of immunostaining of tau protein and βIII tubulin in cerebral organoids in Experimental Example 6.
Fig. 8A is an enlarged staining image of tau protein in the central part of the cerebral organoids of Fig. 7B.
Fig. 8B is an enlarged staining image of tau protein in the central part of the cerebral organoids of Fig. 7C.
Fig. 9A is a fluorescence photomicrograph showing results of immunostaining of cerebral organoids with an MC1 antibody in Experimental Example 6.
Fig. 9B is a fluorescence photomicrograph showing results of immunostaining of cerebral organoids with an MC1 antibody in Experimental Example 6.
Fig. 9C is a fluorescence photomicrograph showing results of immunostaining of cerebral organoids with an MC1 antibody in Experimental Example 6.
Fig. 10A is a fluorescence photomicrograph showing results of immunostaining of MAP2 and GFAP in cerebral organoids in Experimental Example 6.
Fig. 10B is a fluorescence photomicrograph showing results of immunostaining of MAP2 and GFAP in cerebral organoids in Experimental Example 6.
Fig. 10C is a fluorescence photomicrograph showing results of immunostaining of MAP2 and GFAP in cerebral organoids in Experimental Example 6.
Fig. 11A is a fluorescence photomicrograph showing results of staining of cerebral organoids with BTA-1 in Experimental Example 6.
Fig. 11B is a fluorescence photomicrograph showing results of staining of cerebral organoids with BTA-1 in Experimental Example 6.
Fig. 11C is a fluorescence photomicrograph showing results of staining of cerebral organoids with BTA-1 in Experimental Example 6.
Fig. 12A is a photographic image showing results of detecting tau protein and phosphorylated tau protein in cerebral organoids by Western blotting in Experimental Example 6.
Fig. 12B is a photographic image showing results of detecting tau protein and phosphorylated tau protein in cerebral organoids by Western blotting in Experimental Example 6.
Fig. 12C is a photographic image showing results of detecting tau protein and phosphorylated tau protein in cerebral organoids by Western blotting in Experimental Example 6.
Fig. 12D is a graph showing results of calculating the ratio of the phosphorylated tau protein to the total tau proteins based on Fig. 12B and Fig. 12C.
Fig. 13A is a fluorescence photomicrograph showing results of immunostaining of tau protein and HuC/D in cerebral organoids in Experimental Example 6.
Fig. 13B is a fluorescence photomicrograph showing results of immunostaining of tau protein and HuC/D in cerebral organoids in Experimental Example 6.
Fig. 14A is fluorescence photomicrographs showing results of immunostaining of tau protein and CTIP2 in cerebral organoids in Experimental Example 6.
Fig. 14B is enlarged staining images of Fig. 14A.
Fig. 15A is a fluorescence photomicrograph showing results of immunostaining of tau protein and synaptophysin in cerebral organoids in Experimental Example 6.
Fig. 15B is a fluorescence photomicrograph showing results of immunostaining of tau protein and synaptophysin in cerebral organoids in Experimental Example 6.
Fig. 15C is a fluorescence photomicrographs showing results of immunostaining of tau protein and synaptophysin in cerebral organoids in Experimental Example 6.
Fig. 16A is a fluorescence photomicrograph showing results of immunostaining of tau protein and gammaH2A.X in cerebral organoids in Experimental Example 6.
Fig. 16B is a fluorescence photomicrograph showing results of immunostaining of tau protein and gammaH2A.X in cerebral organoids in Experimental Example 6.
Fig. 16C is a fluorescence photomicrographs showing results of immunostaining of tau protein and gammaH2A.X in cerebral organoids in Experimental Example 6.
Fig. 17A is a fluorescence photomicrograph showing results of immunostaining of tau protein and BNIP3 in cerebral organoids in Experimental Example 6.
Fig. 17B is a fluorescence photomicrograph showing results of immunostaining of tau protein and BNIP3 in cerebral organoids in Experimental Example 6.
Fig. 18A is a fluorescence photomicrograph showing results of immunostaining of phosphorylated tau protein and HuC/D, and GFAP in cerebral organoids in Experimental Example 7.
Fig. 18B is a fluorescence photomicrograph showing results of immunostaining of phosphorylated tau protein and HuC/D, and GFAP in cerebral organoids in Experimental Example 7.
Fig. 18C is a fluorescence photomicrograph showing results of immunostaining of phosphorylated tau protein and HuC/D, and GFAP in cerebral organoids in Experimental Example 7.
Fig. 19A is a fluorescence photomicrograph showing results of immunostaining of cerebral organoids with an MC1 antibody, an anti-HuC/D antibody, and anti-GFAP antibody in Experimental Example 8.
Fig. 19B is a fluorescence photomicrograph showing results of immunostaining of cerebral organoids with an MC1 antibody, an anti-HuC/D antibody, and anti-GFAP antibody in Experimental Example 8.
Fig. 19C is a fluorescence photomicrograph showing results of immunostaining of cerebral organoids with an MC1 antibody, an anti-HuC/D antibody, and anti-GFAP antibody in Experimental Example 8.

### Description of Embodiments

### [Production method for cerebral organoid having amyloid plaque]

In one embodiment, the present invention provides a production method for a cerebral organoid having amyloid plaques, the method including a step (a) of forming, in the presence of a SMAD inhibitor, an embryoid body from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a glycogen synthase kinase 3β (GSK3β) inhibitor to form an organoid; and a step (c) of removing the organoid after the step (b) from the extracellular matrix and subjecting the removed organoid to stirring culture in a medium, in which at least a part of the step (c) is carried out in the presence of leukemia inhibitory factor (LIF).

As will be described later in Examples, according to the production method of the present embodiment, it is possible to efficiently form a cerebral organoid having amyloid plaques.

In the present specification, examples of the pluripotent stem cell include an embryonic stem cell (an ES cell) and an induced pluripotent stem cell (an iPS cell). The pluripotent stem cell is preferably a human cell. Examples of the Alzheimer's disease-related gene include a presenilin 1 (PS1) gene, a presenilin 2 (PS2) gene, and a β-amyloid precursor protein (APP) gene.

The NCBI accession number of the genomic DNA of the human PS1 gene is NC 000014.9. The NCBI accession number of the genomic DNA of the human PS2 gene is NC_000001.11. The NCBI accession number of the genomic DNA of the human APP gene is NC 000021.9.

Examples of the pluripotent stem cell having a mutation in the Alzheimer's disease-related gene include a pluripotent stem cell having a mutation in the Alzheimer's disease-related gene that leads to the development of Alzheimer's disease.

Examples of the mutation that leads to the development of Alzheimer's disease include a gene mutation in the PS1 gene, which causes an amino acid mutation (A246E) from alanine to glutamic acid in the 246th amino acid of the PS 1 protein, a gene mutation in the PS2 gene, which causes an amino acid mutation (N141I) from asparagine to isoleucine in the 141st amino acid of the PS2 protein, and a duplication of the APP gene, but are not limited thereto.

The mutation that leads to the development of Alzheimer's disease may be a mutation artificially introduced by genome editing or the like. Alternatively, a pluripotent stem cell prepared from a cell derived from an Alzheimer's disease patient may be used as the pluripotent stem cell having a mutation in the Alzheimer's disease-related gene.

Hereinafter, each step of the production method of the present embodiment will be described. First, in the step (a), a pluripotent stem cell is allowed to form an embryoid body in the presence of a SMAD inhibitor. The step (a) is preferably carried out for about 7 days. Through this step, it is possible to induce a pluripotent stem cell to differentiate into a neural cell. As the SMAD inhibitor, it is preferable to use a BMP inhibitor and a TGF-β inhibitor in combination.

As the BMP inhibitor, dorsomorphin (CAS number: 866405-64-3), DMH1 (CAS number: 1206711-16-1), LDN-193189 (CAS number: 1062368-24-4), or the like can be used. One kind may be used alone, or two or more kinds thereof may be used in combination. The amount of the BMP inhibitor added to the medium is, for example, about 1 to 3 µM.

Examples of the TGF-β inhibitor include A83-01 (CAS number: 909910-43-6), SB-431542 (CAS number: 301836-41-9), and RepSox (CAS number: 446859-33-2). One kind may be used alone, or two or more kinds thereof may be used in combination. The amount of the TGF-β inhibitor added to the medium is, for example, about 1 to 3 µM.

In the production method of the present embodiment, it is preferable that all the steps be carried out in a feeder-free manner without using feeder cells. That is, the pluripotent stem cell having a mutation in the Alzheimer's disease-related gene is preferably a pluripotent stem cell cultured in a feeder-free manner, and the steps (a) to (c) are also preferably carried out in a feeder-free manner. This simplifies the culture operation and makes it possible to prevent feeder cells from being mixed with the cerebral organoid.

Before the step (a), a step of culturing the pluripotent stem cell in the presence of less than 100 ng/mL of fibroblast growth factor-2 (FGF2) may be further carried out. The amount of FGF2 added to the medium is preferably less than 100 ng/mL, and it may be, for example, 50 ng/mL or 10 ng/mL.

FGF2 may be derived from a human or a mouse; however, it is preferably derived from a human.

In a case of culturing a pluripotent stem cell in a feeder-free manner, 100 ng/mL of FGF2 is generally added to the medium. On the other hand, the inventors have revealed that in a case where the amount of FGF2 added to the medium is reduced, the efficiency of producing cerebral organoids having amyloid plaques is increased.

In a case where the amount of FGF2 added is too small, the pluripotent stem cell may differentiate. In addition, in a case where the amount of FGF2 added is too large, it tends to be difficult to obtain a neuroepithelial-like structure.

Further, for example, in a case where the amount of FGF2 added to the medium is set to 10 ng/mL, it becomes difficult to maintain the pluripotent stem cell when the culture is continued for about 3 weeks or more. As a result, in a case where the pluripotent stem cell is cultured in a feeder-free manner and in the presence of 10 ng/mL FGF2, the culture period is preferably 4 weeks or less.

Subsequently, in the step (b), the embryoid body after the step (a) is embedded in an extracellular matrix and three-dimensionally cultured in the presence of a SMAD inhibitor and a glycogen synthase kinase 3β (GSK3β) inhibitor to form an organoid. Here, it is preferable that the embryoid body be embedded in the extracellular matrix without being dispersed. The dispersion method for the embryoid body is not particularly limited, and examples thereof include a physical method and an enzyme treatment method. However, an enzyme treatment method is preferable from the viewpoint that cells are not damaged. As the enzyme used in the enzyme treatment method, TrypLE Express (Thermo Fisher Scientific, Inc.), TrypLE Select (Thermo Fisher Scientific, Inc.), trypsin, collagenase, dispase I, or the like can be used. The step (b) is preferably carried out for about 7 days.

Examples of the extracellular matrix include type IV collagen, laminin, heparan sulfate proteoglycan, and entactin. As the extracellular matrix, for example, a commercially available product such as Matrigel (manufactured by Corning Incorporated) may be used.

As the SMAD inhibitor, it is preferable to use the above-described TGF-β inhibitor. One kind of SMAD inhibitor may be used alone in the step (b), and two or more kinds thereof are used in combination in the step (a). The amount of the SMAD inhibitor added to the medium is, for example, about 1 to 5 µM. Among the above, it is preferable to use SB-431542 in the step (b).

Examples of the GSK3β inhibitor include CHIR99021 (CAS number: 252917-06-9), kenpaullone (CAS number: 142273-20-9), and 6-bromoindirubin-3'-oxime (CAS number: 029-16241). The amount of the GSK3β inhibitor added to the medium is, for example, about 1 to 5 µM. Among the above, it is preferable to use CHIR99021.

Subsequently, in the step (c), the organoid after the step (b) is removed from the extracellular matrix and subjected to stirring culture in the medium. The stirring culture is preferably carried out using a bioreactor. As the bioreactor, a commercially available one can be used.

The step (c) is preferably carried out for 100 days or more. As will be described later in Examples, the formation of amyloid plaques was not observed as a result of analyzing cerebral organoids on the 70th day after the start of the step (c), whereas the formation of a large number of amyloid plaques was confirmed when cerebral organoids subjected to the step (c) for 100 days or more were analyzed.

It is preferable to add leukemia inhibitory factor (LIF) to the medium in at least a part of the step (c). The amount of LIF added is, for example, about 5 to 50 ng/mL. The addition of LIF to the medium may be carried out only in a part of the step (c) or in the whole step (c). LIF may be derived from a human or a mouse; however, it is preferably derived from a human. The inventors have revealed that the efficiency of producing cerebral organoids having amyloid plaques is increased by carrying out stirring culture in the presence of LIF.

It is preferable that at least a part of the step (c) be carried out in the presence of more than 20% by volume of oxygen. The term "more than 20% by volume of oxygen" is an oxygen concentration higher than the oxygen concentration (about 20% by volume) in normal air. The oxygen concentration in the step (c) is preferably more than 20% by volume, and is, for example, 30% by volume, 40% by volume, or 50% by volume. The inventors have revealed that the efficiency of producing cerebral organoids having amyloid plaques is increased by carrying out stirring culture under a higher oxygen concentration than usual.

The period in which the oxygen concentration is high may be, for example, a period from about 7 days after the start of the step (c) to the end of the culture, may be a period from about 14 days after the start of the step (c) to the end of the culture, and may be a period from about 21 days after the start of the step (c) to the end of the culture.

### [Cerebral organoid having amyloid plaques]

In one embodiment, the present invention provides a cerebral organoid having amyloid plaques of which the diameters are 20 µm or more. In the cerebral organoid of the present embodiment, the diameter of the amyloid plaque may be more than 20 µm, 25 µm or more, 30 µm or more, 35 µm or more, or 40 µm or more. The diameter of the amyloid plaque can be measured by immunostaining a tissue section with an anti-amyloid β antibody and observing the cross-sectional shape of the amyloid plaque under a microscope. In a case where the cross-sectional shape of the amyloid plaque is not a perfect circle, a perfect circle having the same area as the cross-sectional shape of the amyloid plaque is assumed, and the diameter thereof may be taken as the diameter of the amyloid plaque.

The cerebral organoid of the present embodiment is preferably derived from a human. In the related art, it has been difficult to efficiently prepare a human cerebral organoid having amyloid plaques. On the other hand, since the cerebral organoid of the present embodiment can be efficiently prepared, it is useful as a model for studying the mechanism of the development of Alzheimer's disease or for studying the preventive and therapeutic methods for Alzheimer's disease. The cerebral organoid of the present embodiment can be produced by the production method described above.

In the cerebral organoid of the present embodiment, it is preferable that the average of the proportion of the area per amyloid plaque having a diameter of 20 µm or more to the total area of the cross section of the cerebral organoid be 0.1% or more.

In the cerebral organoid of the present embodiment, the number of amyloid plaques having a diameter of 20 µm or more is preferably 2 or more per cerebral organoid.

In addition, in the cerebral organoid of the present embodiment, the molar ratio of the expression amount of amyloid β42 to the expression amount of amyloid β40 (the expression amount of amyloid β42/the expression amount of amyloid β40) is preferably 0.15 or more. The amino acid sequence of amyloid β40 is shown in SEQ ID NO: 1, and the amino acid sequence of amyloid β42 is shown in SEQ ID NO: 2.

The molar ratio of the expression amount of amyloid β42 to the expression amount of amyloid β40 is more preferably 0.16 or more, still more preferably 0.18 or more, and particularly preferably 0.2 or more.

The cerebral organoid having any one of the above-described characteristics is conceived to reflect the pathophysiology of Alzheimer's disease and thus is useful as a model for Alzheimer's disease.

As will be described later, the cerebral organoid of the present embodiment can be used for screening for a therapeutic drug for Alzheimer's disease. Accordingly, in one embodiment, the present invention provides a screening kit for a therapeutic drug for Alzheimer's disease, which includes a cerebral organoid having amyloid plaques. The cerebral organoid having amyloid plaques is the same as those described above.

### [Screening method for therapeutic drug for Alzheimer's disease]

In one embodiment, the present invention provides a screening method for a therapeutic drug for Alzheimer's disease, the screening method including a step of culturing the above-described cerebral organoid having amyloid plaques in the presence of a test substance; and a step of measuring the sizes of amyloid plaques of the cerebral organoid, in which the reduction of the sizes of the amyloid plaques indicates that the test substance is a therapeutic drug for Alzheimer's disease.

The test substance is not particularly limited, and examples thereof include a natural compound library, a synthetic compound library, an existing drug library, and a metabolite library.

The size of the amyloid plaque can be measured, for example, based on a staining image which is obtained by subjecting the cerebral organoid to immunostaining with an anti-amyloid β antibody. Alternatively, it can be measured based on a staining image which is obtained by staining the cerebral organoid with a reagent capable of staining amyloid plaques, such as 2-(4'-methylaminophenyl)benzothiazole (BTA-1).

In a case where the size of the amyloid plaque of the cerebral organoid is reduced in the presence of a test substance, the test substance can be said to be a therapeutic drug for Alzheimer's disease.

For example, in a case where the size of the amyloid plaque of the cerebral organoid in the presence of a test substance is reduced as compared with the size of the amyloid plaque of the cerebral organoid in the absence of the test substance, the test substance can be said to be a therapeutic drug for Alzheimer's disease.

Alternatively, in a case where the size of the amyloid plaque of the cerebral organoid after the administration of a test substance is reduced as compared with the size of the amyloid plaque of the cerebral organoid before the administration of the test substance, the test substance can be said to be a therapeutic drug for Alzheimer's disease.

According to the screening method of the present embodiment, a therapeutic drug for Alzheimer's disease can be screened. It can be said that the therapeutic drug for Alzheimer's disease obtained by the screening method of the present embodiment is a drug that reduces or eliminates the formed amyloid plaques.

### [Screening method for prophylactic drug for Alzheimer's disease]

In one embodiment, the present invention provides a screening method for a prophylactic drug for Alzheimer's disease, the method including a step (a) of forming, in the presence of a SMAD inhibitor, an embryoid body from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a GSK3β inhibitor to form an organoid; a step (c) of removing the organoid after the step (b) from the extracellular matrix and subjecting the removed organoid to stirring culture in a medium to obtain a cerebral organoid, at least a part of the step (c) being carried out in the presence of a test substance; and a step (d) of measuring the sizes of amyloid plaques of the cerebral organoid, after carrying out the step (c) for 100 days or more, in which in the step (d), a reduction of the sizes of the amyloid plaques as compared with a control indicates that the test substance is a prophylactic drug for Alzheimer's disease.

In the screening method of the present embodiment, the steps (a) and (b) are the same as the steps (a) and (b) in the above-described production method for a cerebral organoid having amyloid plaques. In the screening method of the present embodiment, the step (c) is the same as the above-described step (c) in the production method for a cerebral organoid having amyloid plaques; however, it is different from the above-described production method in that at least a part of the step (c) is carried out in the presence of the test substance.

The test substance is the same as that described above in the screening method for a therapeutic drug for Alzheimer's disease.

In the screening method of the present embodiment, the size of the amyloid plaque of the cerebral organoid is measured in the step (d). The size of the amyloid plaque can be measured in the same manner as described above in the screening method for a therapeutic drug for Alzheimer's disease.

In the step (d), in a case where the size of the amyloid plaque of the cerebral organoid cultured in the presence of a test substance is reduced as compared with the control, it can be said that the test substance is a prophylactic drug for Alzheimer's disease. Here, examples of the control include a cerebral organoid cultured in the absence of the test substance.

According to the screening method of the present embodiment, a prophylactic drug for Alzheimer's disease can be screened. It can be said that the prophylactic drug for Alzheimer's disease obtained by the screening method of the present embodiment is a drug that suppresses or prevents the formation of amyloid plaque by being administered before the formation thereof.

### [Screening method for prophylactic drug or therapeutic drug for Alzheimer's disease]

In one embodiment, the present invention provides a screening method for a prophylactic drug or a therapeutic drug for Alzheimer's disease, the method including a step (a) of forming, in the presence of a SMAD inhibitor, an embryoid body from a pluripotent stem cell having a mutation in an Alzheimer's disease-related gene; a step (b) of embedding the embryoid body after the step (a) in an extracellular matrix and three-dimensionally culturing the embedded embryoid body in the presence of a SMAD inhibitor and a GSK3β inhibitor to form an organoid; a step (c) of removing the organoid after the step (b) from the extracellular matrix and subjecting the removed organoid to stirring culture in a medium to obtain a cerebral organoid, at least a part of the step (c) being carried out in the presence of a test substance; and a step (d') of quantifying expression amounts of amyloid β40 and amyloid β42 expressed by the cerebral organoid of the step (c), in which in the step (d'), a reduction of a ratio of an expression amount of amyloid β42 to an expression amount of amyloid β40 as compared with a control indicates that the test substance is a prophylactic drug or therapeutic drug for Alzheimer's disease.

In the screening method of the present embodiment, the steps (a) to (c) are the same as the above-described steps (a) to (c) in the screening method for a prophylactic drug for Alzheimer's disease. In addition, the test substance is the same as that described above in the screening method for a therapeutic drug for Alzheimer's disease.

In the screening method of the present embodiment, the expression amounts of amyloid β40 and amyloid β42 expressed by the cerebral organoid are quantified in the step (d'). The expression amounts of amyloid β40 and amyloid β42 may be quantified by, for example, the same ELISA method as described later in Examples. Specifically, the cerebral organoid to be measured may be transferred to a tube or a well of a microplate and cultured for about 24 to 48 hours, and then the amyloid β40 and amyloid β42 secreted in the medium may be measured.

In the step (d'), in a case where the ratio of the expression amount of amyloid β42 to the expression amount of amyloid β40 expressed by the cerebral organoid cultured in the presence of the test substance is reduced as compared with the control, it can be said that the test substance is a prophylactic drug or therapeutic drug for Alzheimer's disease. Here, examples of the control include a cerebral organoid cultured in the absence of the test substance.

In the step (d'), the ratio of the expression amount of amyloid β42 to the expression amount of amyloid β40 may be, for example, a molar ratio. For example, in a case where the molar ratio (the expression amount of amyloid β42/the expression amount of amyloid β40) is reduced to less than 0.2, for example, less than 0.15 by the administration of a test substance, the test substance can be said to be a prophylactic drug or therapeutic drug for Alzheimer's disease. That is, according to the screening method of the present embodiment, a prophylactic drug or therapeutic drug for Alzheimer's disease can be screened.

### Examples

Next, the present invention will be described in more detail by showing Examples, however, the present invention is not limited to Examples below.

### [Experimental Example 1]

### (Preparation of cerebral organoid 1)

Pluripotent stem cells were cultured to prepare cerebral organoids. As the pluripotent stem cells, 414C2, 201B7, and RPC771, which are wild-type human iPS cell lines, KhES1, which is a wild-type human ES cell line, and PS1-2 and PS2-2, which are human iPS cell lines derived from Alzheimer's disease patients, were used.

The PS 1-2 cell has been revealed to have a gene mutation in the PS1 gene, which causes an amino acid mutation (A246E) from alanine to glutamic acid in the 246th amino acid of the PS 1 protein. In addition, the PS2-2 cell has been revealed to have a gene mutation in the PS2 gene, which causes an amino acid mutation (N141I) from asparagine to isoleucine in the 141 st amino acid of the PS2 protein.

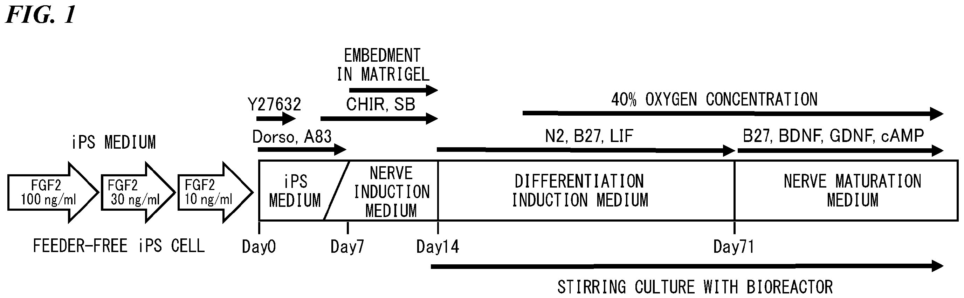

Fig. 1 is a diagram showing a schedule for preparing cerebral organoids. Each kind of pluripotent stem cell was cultured in a feeder-free manner in which feeder cells were not used. First, each kind of cell was cultured in a medium containing 10 ng/mL FGF2 for 1 to 3 weeks.

Subsequently, on 0th day of culture (day 0), each kind of cell dissociated into a single cell was suspended in an iPS medium containing 2 µM dorsomorphin (Sigma-Aldrich Co., LLC) and 2 µM A83-01 (Tocris Biosciences), 10 µM Y27632 (Nacalai Tesque, Inc.) but not containing FGF2, and seeded in a 96-well plate. On the first day of culture (day 1), the same amount of a medium obtained by excluding Y27632 from the above medium was added thereto. Half of the amount of the medium was replaced on the third day of culture (day 3). In Fig. 1, "Dorso" indicates dorsomorphin, and "A83" indicates A83-01.

Subsequently, on the 5th to 6th days of culture (days 5 to 6), half the amount of the medium was replaced with an induction medium. The composition of the induction medium was DMEM/F12, GlutaMAX (Thermo Fisher Scientific, Inc.), 1 µM CHIR99021 (Cellagen Technology LLC), 1 µM SB-431542 (Cellagen Technology LLC), 1 × N2 supplement (Thermo Fisher Scientific, Inc.), 1 × NEAA (Thermo Fisher Scientific, Inc.), and 1 × penicillin/streptomycin. In Fig. 1, "CHIR" indicates CHIR99021, and "SB" indicates SB-431542.

Subsequently, on the 7th day of culture (day 7), each kind of cell was embedded in Matrigel (BD Bioscience) and cultured in the induction medium for another 6 days. Half of the amount of the medium was changed every other day.

Subsequently, on the 14th day of culture (day 14), the Matrigel was mechanically dissociated to remove the formed organoids by pipetting using a 5 mL pipette. Subsequently, the organoid was suspended in a differentiation medium, which was subsequently placed in a bioreactor (ABLE Corporation) and subjected to stirring culture. The composition of the differentiation medium was DMEM/F12, GlutaMAX (Thermo Fisher Scientific, Inc.), 1 × N2 supplement (Thermo Fisher Scientific, Inc.), 1 × B27 supplement (Thermo Fisher Scientific, Inc.), 2.5 µg mL insulin (Sigma-Aldrich Co., LLC) 0.1 mM 2-mercaptoethanol, 1 × NEAA (Thermo Fisher Scientific, Inc.), 1 × penicillin/streptomycin, and 10 ng/mL LIF (Merck Millipore). The medium was changed every two or three days. In Fig. 1, "N2" indicates an N2 supplement and "B27" indicates a B27 supplement.

From the 28th day of culture (day 28), the oxygen concentration in the incubator was set to 40% by volume.

From the 71st day of culture (day 71), the differentiation medium was replaced with a maturation medium, and stirring culture was continued. The composition of the maturation medium was Neurobasal Plus (Thermo Fisher Scientific, Inc.), 1 × B27 supplement (Thermo Fisher Scientific, Inc.), 20 ng/mL BDNF (PeproTech, Inc.), 20 ng/mL GDNF (PeproTech, Inc.), 0. 5 mM cAMP (Sigma-Aldrich Co., LLC) 1 × GlutaMAX (Thermo Fisher Scientific, Inc.), 0.2 mM ascorbic acid (Sigma-Aldrich Co., LLC), 1 × Antibiotic-Antimycotic (Thermo Fisher Scientific, Inc.), and 10 ng/mL LIF (Merck Millipore). The medium was changed every two or three days.

### [Experimental Example 2]

### (Detection of amyloid plaque 1)

Amyloid plaques in the cerebral organoids prepared in Experimental Example 1 were detected. On the 120th day of culture (day 120, 106 days after the start of stirring culture), each cerebral organoid was removed and fixed with 4% paraformaldehyde. Subsequently, the fixed organoid was embedded in a resin to prepare a frozen section.

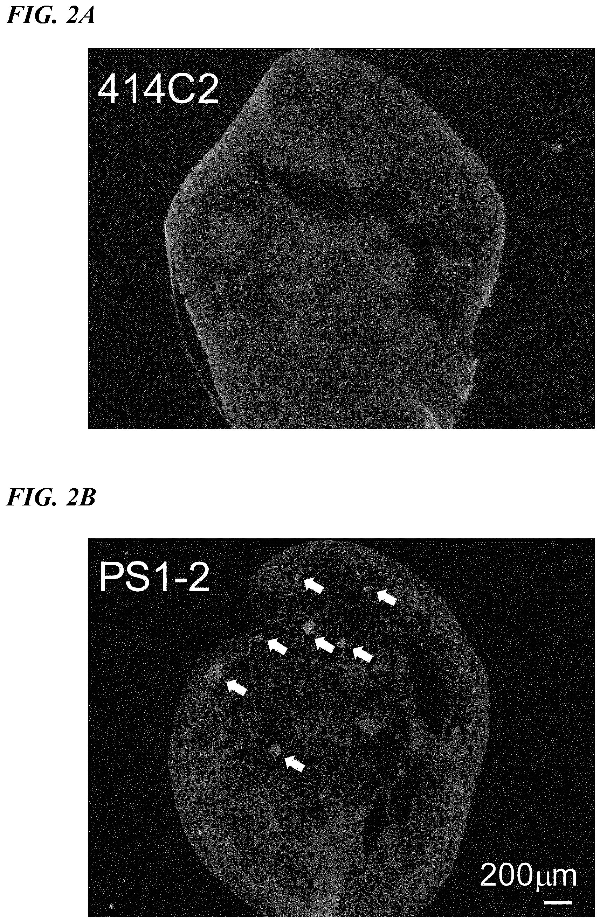

Subsequently, each frozen section was immunostained with an anti-amyloid β antibody (clone 6E10, BioLegend) to detect amyloid plaques. Fig. 2Ais a representative photographic image showing the result of the cerebral organoid on the 120th day of culture, which was prepared from the wild-type human iPS cell line 414C2. In addition, Fig. 2B is a representative photographic image showing the result of the cerebral organoid on the 120th day of culture, which was prepared from the human iPS cell line PS 1-2 derived from an Alzheimer's disease patient. In Fig. 2B, arrows indicate amyloid plaques having a diameter of 20 µm or more.

From the above results, it was revealed that amyloid plaques were detected with good reproducibility in the cerebral organoids prepared from iPS cells derived from Alzheimer's disease patients.

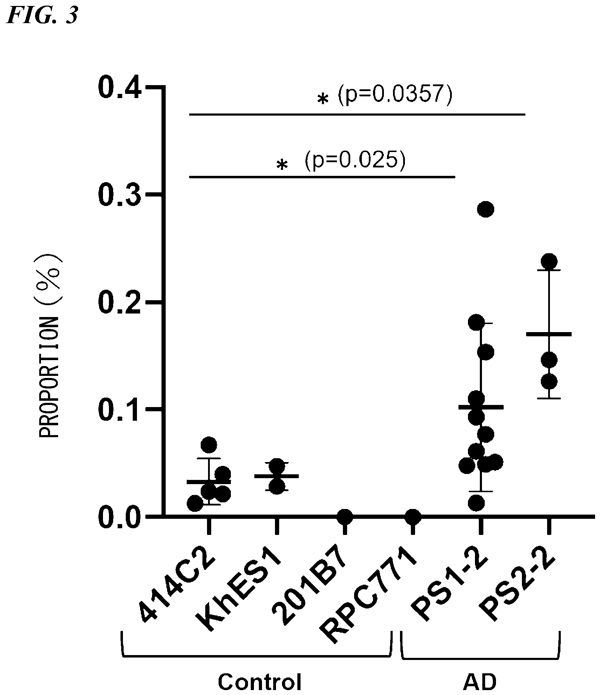

Fig. 3 is a graph showing the proportion (%) of the area per amyloid plaque having a diameter of 20 µm or more with respect to the total area of the section (the cross section) in the cerebral organoid 120 days after culture, which was prepared from each pluripotent stem cell. Three organoids were analyzed for each of the cerebral organoids derived from 414C2, KhES1, PS1-2, and PS2-2. In addition, two organoids were analyzed for each of the cerebral organoids derived from 201B7 and RPC771. In Fig. 3, "Control" indicates control iPS cells or control cerebral organoids derived from ES cells, and "AD" indicates cerebral organoids derived from iPS cells derived from Alzheimer's disease patients. In addition, "^{∗}" indicates that there is a significant difference at p <0.05 as a result of the Mann-Whitney test.

As a result of the above analysis, in the cross section of the cerebral organoid from PS 1-2 or PS2-2, the average of the proportion of the area per amyloid plaque having a diameter of 20 µm or more with respect to the total area of the cross section was 0.1% or more.

As a result of carrying out the same examination on the 84th day of culture (day 84, 70 days after the start of stirring culture), amyloid plaques were not detected in the cerebral organoids derived from any cells. Accordingly, it was revealed that in order to form amyloid plaques, it is necessary to culture for about 100 days or more after the start of stirring culture.

### [Experimental Example 3]

### (Detection of amyloid plaque 2)

Using 2-(4'-methylaminophenyl)benzothiazole (BTA-1, Sigma-Aldrich Co., LLC) instead of the anti-amyloid β antibody, amyloid plaques in the cerebral organoid prepared in Experimental Example 1 were detected. BTA-1 is a derivative of thioflavin-T and is a compound that exhibits a high affinity to deposits of amyloid β peptide, which is about 50 times higher than that of thioflavin-T.

On the 120th day of culture (day 120, 106 days after the start of stirring culture), each cerebral organoid was removed and fixed with 4% paraformaldehyde. Subsequently, the fixed organoid was embedded in a resin to prepare a frozen section.

Subsequently, each frozen section was stained with BTA-1 to detect amyloid plaques. In addition, for comparison, a control human cerebral tissue section (26 years old) and a cerebral tissue section of an Alzheimer's disease patient (73 years old) were also stained with BTA-1 in the same manner.

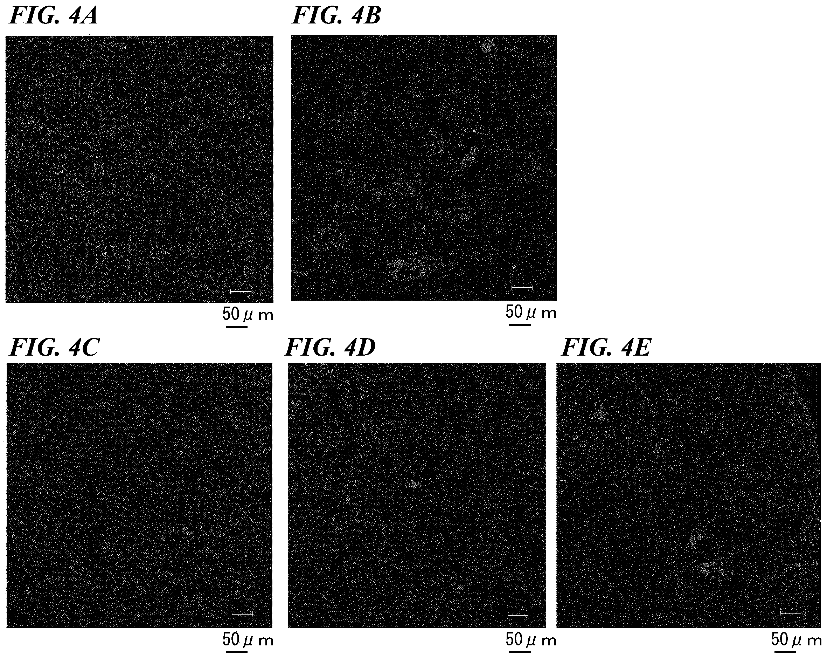

Fig. 4A is a representative photographic image showing the result of the control (26 years old) human cerebral tissue. Fig. 4B is a representative photographic image showing the result of the cerebral tissue of the Alzheimer's disease patient (73 years old). Fig. 4C is a representative photographic image showing the result of the cerebral organoid on the 120th day of culture, which was prepared from the wild-type human iPS cell line 414C2. Fig. 4D is a representative photographic image showing the result of the cerebral organoid on the 120th day of culture, which was prepared from the human iPS cell line PS 1-2 derived from an Alzheimer's disease patient. Fig. 4E is a representative photographic image showing the result of the cerebral organoid on the 120th day of culture, which was prepared from the human iPS cell line PS2-2 derived from an Alzheimer's disease patient.

As a result of the above staining, no amyloid plaque was detected in the control (26 years old) human cerebral tissue and the 414C2-derived cerebral organoid. On the other hand, amyloid plaques were detected in the cerebral tissue of the Alzheimer's disease patient (73 years old), the cerebral organoid on the 120th day of culture, which had been prepared from PS 1-2, and the cerebral organoid on the 120th day of culture, which had been prepared from PS2-2.

### [Experimental Example 4]

### (Quantification of expression amounts of amyloid β40 and amyloid β42)

Each of the amyloid β40 peptide and the amyloid β42 peptide secreted in the culture supernatant of the cerebral organoids on the 120th day of culture (day 120, 106 days after the start of stirring culture), which had been prepared in the same manner as in Experimental Example 1, was quantified by the ELISA method using a commercially available kit (FUJIFILM Wako Pure Chemical Corporation).

Specifically, one organoid of the cerebral organoids on the 118th day of culture (day 118, 104 days after the start of stirring culture) was transferred to each well of a 48-well plate and cultured for another 2 days. As the cerebral organoid, cerebral organoids prepared from 414C2, which is a wild-type human iPS cell line, KhES 1, which is a wild-type human ES cell line, and PS1-2 and PS2-2, which are human iPS cell lines derived from Alzheimer's disease patients, were used.

Subsequently, each of the amyloid β40 peptide and the amyloid β42 peptide, which had been secreted in the culture supernatant on the 120th day of culture (day 120, 106 days after the start of stirring culture), was quantified.

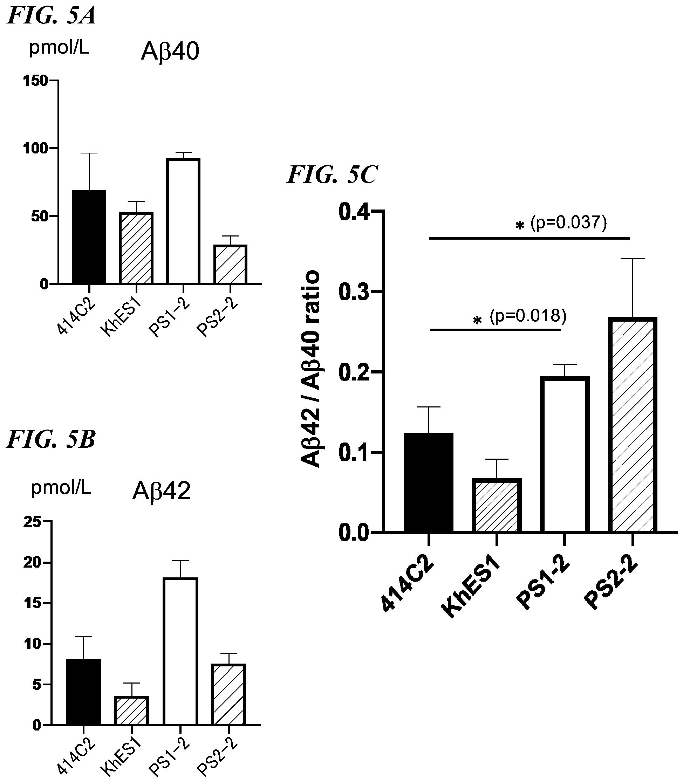

Fig. 5A is a graph showing results of quantifying amyloid β40 (Aβ40). In addition, Fig. 5B is a graph showing results of quantifying amyloid β42 (Aβ42). In addition, Fig. 5C is a graph showing results of calculating the molar ratio of an expression amount of amyloid β42 to an expression amount of amyloid β40 (the expression amount of amyloid β42/the expression amount of amyloid β40) of each cerebral organoid based on the results of Fig. 5A and Fig. 5B. In Fig. 5C, "^{∗}" indicates that there is a significant difference at p <0.05 as a result of the unpaired Student's t-test.

From the above results, it was revealed that in the cerebral organoids prepared from human iPS cell lines (PS 1-2 and PS2-2) derived from Alzheimer's disease patients, the molar ratio of the expression amount of amyloid β42 to the expression amount of amyloid β40 (the expression amount of amyloid β42/the expression amount of amyloid β40) is significantly high as compared with those of the control cerebral organoids (414C2 and KhES1). It is known that in a case where the molar ratio of the expression amount of amyloid β42 to the expression amount of amyloid β40 is high, amyloid plaques are easily formed, which is supported by the results of this Experimental Example.

### [Experimental Example 5]

### (Preparation of cerebral organoid 2)

Cerebral organoids were prepared from PS 1-2 and PS2-2, which are human iPS cell lines derived from Alzheimer's disease patients. The cerebral organoids were prepared in the same manner as in Experimental Example 1 except that each kind of cell was cultured in the presence of 100 ng/mL FGF2 for 1 to 3 weeks before the first day of culture (day 1).

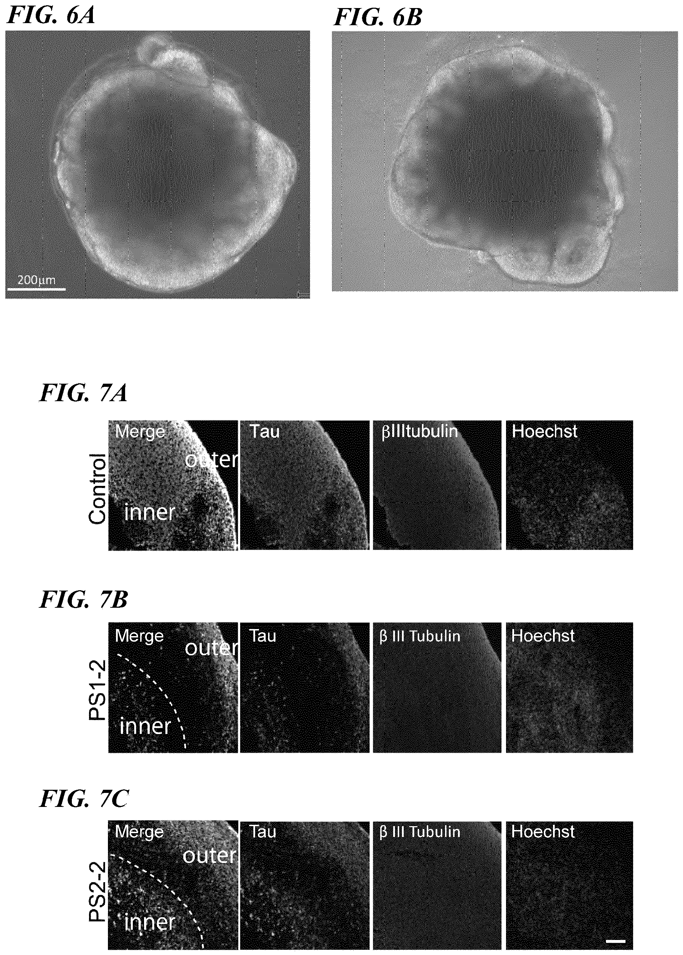

From the above results, it was revealed that in a case where iPS cells cultured in the presence of 100 ng/mL FGF2 are induced to differentiate, the efficiency of forming a neuroepithelial-like structure after being embedded in Matrigel is decreased as compared with a case where iPS cells cultured in the presence of 10 ng/mL FGF2 were induced to differentiate. Fig. 6A is a photographic image of the cerebral organoid on the 14th day of culture (day 14), which had been obtained by differentiation induction using iPS cells cultured in the presence of 100 ng/mL FGF2. Fig. 6B is a photographic image of the cerebral organoid on the 14th day of culture (day 14), which had been obtained by differentiation induction using iPS cells cultured in the presence of 10 ng/mL FGF2. From the above results, it was revealed that a clearer neuroepithelial-like structure is confirmed in Fig. 6B as compared with Fig. 6A.

### [Experimental Example 6]

### (Examination of phenotype of tau protein)

As characteristic pathological changes in Alzheimer's disease, senile plaque deposition, neurofibrillary tangle (NFT), and cerebral atrophy are known. Since NFT is generated by the accumulation of insoluble aggregates of tau in cells, immunostaining was carried out to analyze the phenotype of tau protein in cerebral organoids (PS 1-2 and PS2-2) prepared from human iPS cell lines derived from Alzheimer's disease patients.

### <<Immunostaining of tau protein and βIII tubulin>>

Cerebral organoids prepared in the same manner as in Experimental Example 1 were fixed with 4% paraformaldehyde on the 84th day of culture (day 84, 70 days after the start of stirring culture). Subsequently, the fixed organoids were embedded in an O.C.T. compound (KENIS, Ltd.) to prepare a frozen section.

Subsequently, each frozen section was immunostained with an anti-tau antibody (FUJIFILM Wako Pure Chemical Corporation, Agilent Technologies, Inc.). In addition, immunostaining of βIII tubulin, which is a marker for the immature nerve cell, was also carried out. In addition, nuclei were also stained with Hoechst33342.

Fig. 7A, Fig. 7B and Fig. 7C are fluorescence photomicrographs showing the results of immunostaining. Fig. 7A is a representative photographic image showing the result of the cerebral organoid on the 84th day of culture, which was prepared as a control from wild-type human iPS cell line 414C2. In addition, Fig. 7B is a representative photographic image showing the result of the cerebral organoid on the 84th day of culture, which was prepared from the human iPS cell line PS 1-2 derived from an Alzheimer's disease patient. In addition, Fig. 7C is a representative photographic image showing the result of the cerebral organoid on the 84th day of culture, which was prepared from the human iPS cell line PS2-2 derived from an Alzheimer's disease patient. The scale bar is 100 µm. In addition, in Fig. 7A, Fig. 7B and Fig. 7C, "inner" indicates the central part of the organoid, and "outer" indicates the outer edge part of the organoid.

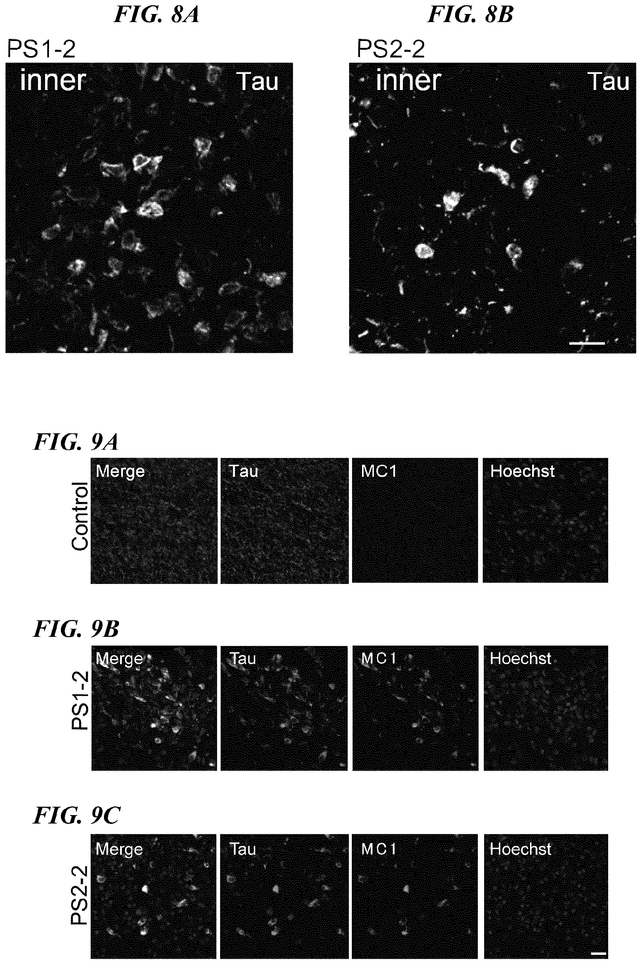

In addition, Fig. 8A and Fig. 8B are enlarged staining images of tau protein in the central part of the organoids in Fig. 7B and Fig. 7C. The scale bar is 20 µm. Fig. 8A is the result of the cerebral organoid prepared from PS1-2, and Fig. 8B is the result of the cerebral organoid prepared from PS2-2.

From the above results, it was revealed that in the cerebral organoid on the 84th day, the expression of tau protein is uniformly observed in the whole organoid in the cerebral organoid prepared from the control (414C2), whereas in the cerebral organoids prepared from PS 1-2 and PS2-2, the tau protein is accumulated in the cell body inside the cerebral organoid.

### <<Immunostaining with MC1 antibody>>

Subsequently, immunostaining was carried out with an MC1 antibody (provided by Dr. Peter Davies) that recognizes an early three-dimensional structural change of tau protein, which is observed in Alzheimer's disease. In addition, nuclei were also stained with Hoechst33342. Fig. 9A, Fig. 9B and Fig. 9C are fluorescence photomicrographs showing the results of immunostaining of the central part of the organoids. Fig. 9A is a representative photographic image showing the result of the cerebral organoid on the 84th day of culture, which was prepared as a control from wild-type human iPS cell line 414C2. In addition, Fig. 9B is a representative photographic image showing the result of the cerebral organoid on the 84th day of culture, which was prepared from the human iPS cell line PS 1-2 derived from an Alzheimer's disease patient. In addition, Fig. 9C is a representative photographic image showing the result of the cerebral organoid on the 84th day of culture, which was prepared from the human iPS cell line PS2-2 derived from an Alzheimer's disease patient. The scale bar is 20 µm.

From the above results, it was revealed that the cells in which the accumulation of tau protein was observed inside the cerebral organoids prepared from PS 1-2 and PS2-2 are stained with the MC1 antibody. This result indicates that the three-dimensional structural change of the accumulated tau protein occurs in these cells.

### «Immunostaining of MAP2 and GFAP»

Subsequently, immunostaining of MAP2, which is a marker for the mature nerve cell, was carried out. In addition, immunostaining of GFAP, which is a marker for the astrocyte, was also carried out. In addition, nuclei were also stained with Hoechst33342.

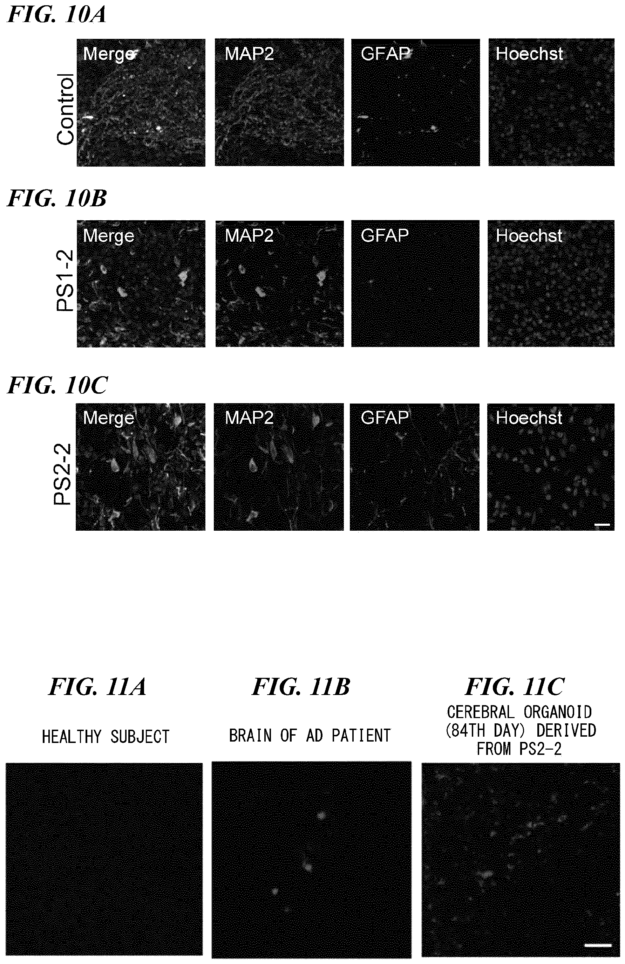

Fig. 10A, Fig. 10B and Fig. 10C are fluorescence photomicrographs showing the results of immunostaining. Fig. 10A is a representative photographic image showing the result of the cerebral organoid on the 84th day of culture, which was prepared as a control from wild-type human iPS cell line 414C2. In addition, Fig. 10B is a representative photographic image showing the result of the cerebral organoid on the 84th day of culture, which was prepared from the human iPS cell line PS1-2 derived from an Alzheimer's disease patient. In addition, Fig. 10C is a representative photographic image showing the result of the cerebral organoid on the 84th day of culture, which was prepared from the human iPS cell line PS2-2 derived from an Alzheimer's disease patient. The scale bar is 20 µm.

From the above results, it was revealed that MAP2, which is a marker for the mature nerve cell, is accumulated in the cell body in the nerve cells inside the cerebral organoid.

### «Staining with BTA-1»

Subsequently, a frozen section of the cerebral organoid prepared from PS2-2 on the 84th day of culture was stained with BTA-1 (Sigma-Aldrich Co., LLC) which is a derivative of thioflavin-T. In addition, for comparison, a cerebral tissue section of a healthy subject and a cerebral tissue section of an Alzheimer's disease patient were also stained with BTA-1.

Fig. 11A, Fig. 11B and Fig. 11C are fluorescence photomicrographs showing the staining results. The scale bar is 50 µm. Fig. 11A is a representative result of the brain of the healthy subject, Fig. 11B is a representative result of the brain of the Alzheimer's disease patient, and Fig. 11C is a representative result of the cerebral organoid on the 84th day of culture, which had been prepared from PS2-2.

From the above results, it was revealed that the cerebral organoid on the 84th day of culture, which had been prepared from PS2-2, is stained with BTA-1 similarly to the brain of the Alzheimer's disease patient.

### «Western blotting of tau protein and phosphorylated tau protein»

Subsequently, the extract of the cerebral organoid on the 84th day of culture, which had been prepared from PS 1-2, was analyzed by Western blotting to detect tau protein and phosphorylated tau protein. In addition, as a control, an extract of a cerebral organoid on the 84th day of culture, which had been prepared from wild-type human iPS cell line 414C2, was analyzed in the same manner.

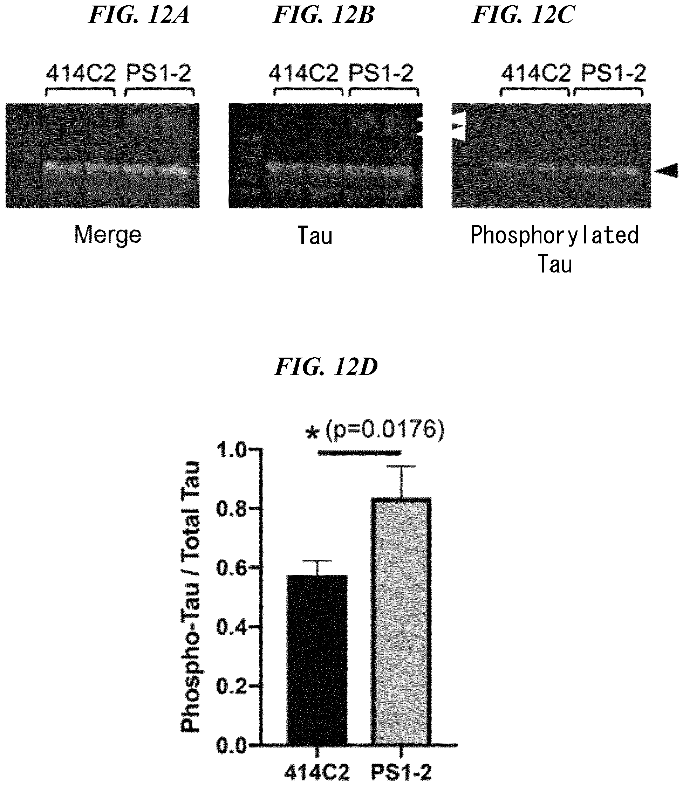

Fig. 12A, Fig. 12B and Fig. 12C are photographic images showing the results of Western blotting. Fig. 12A shows the result obtained by merging Fig. 12B with Fig. 12C. Fig. 12B shows the results of detecting the total tau proteins. Fig. 12C shows the results of detecting phosphorylated tau protein. In addition, Fig. 12D is a graph showing results of calculating the ratio of the phosphorylated tau protein to the total tau proteins based on Fig. 12B and Fig. 12C.

From the above results, it was revealed that in the cerebral organoid on the 84th day of culture, which had been prepared from human iPS cell line PS 1-2 derived from an Alzheimer's disease patient, the proportion of the phosphorylated tau protein in the total tau proteins is high as compared with the control.

### «Immunostaining of HuC/D and CTIP2»

Subsequently, cells in which tau protein is accumulated in the cytoplasm, which are observed in the central part of the cerebral organoid on the 84th day of culture, were immunostained with HuC/D, which is a marker for the nerve cell, and CTIP2, which is a marker for the nerve cell layer.

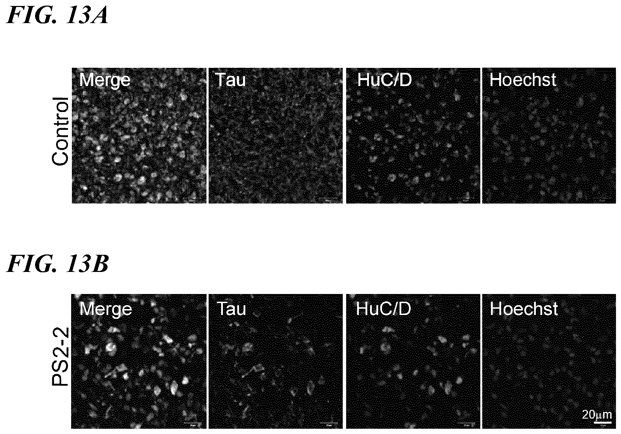

Fig. 13A and Fig. 13B are fluorescence photomicrographs showing results of immunostaining of tau protein and HuC/D. As a control, the cerebral organoid on the 84th day of culture, which had been prepared from the wild-type human iPS cell line 414C2, was also subjected to immunostaining. The scale bar is 20 µm.

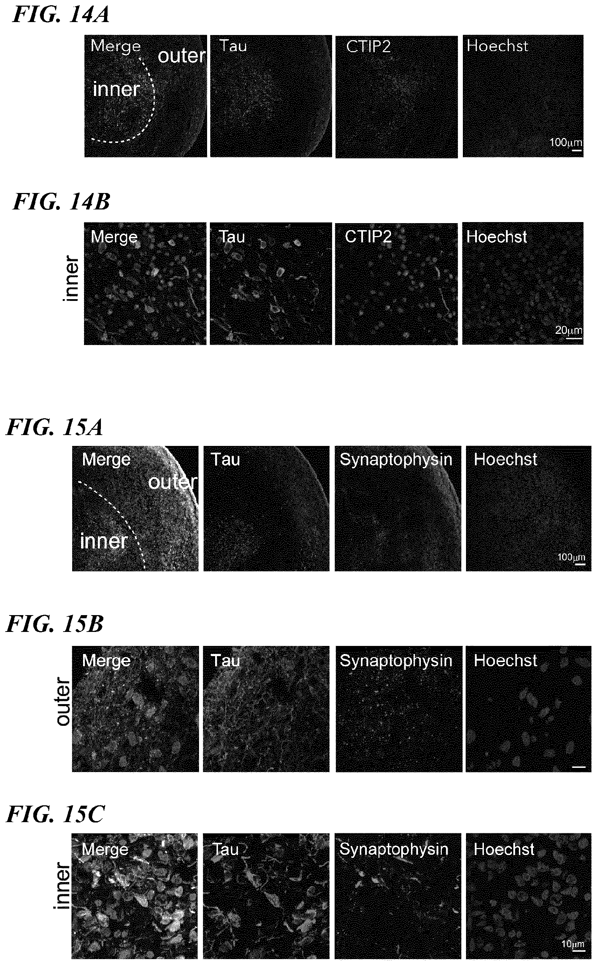

In addition, Fig. 14A is a fluorescence photomicrograph showing results of immunostaining of tau protein and CTIP2 in the cerebral organoid on day 84 of culture, which had been prepared from PS1-2. In Fig. 14A, "inner" indicates the central part of the organoid, and "outer" indicates the outer edge part of the organoid. The scale bar is 100 µm. In addition, Fig. 14B is enlarged staining images in the central part of the organoid of Fig. 14A. The scale bar is 20 µm.

As a result of the above immunostaining, CTIP2-positive cells, which are a marker for the layer 5 nerve cells, were observed in the central part of the cerebral organoid, and some of the cells showed the accumulation of tau protein in the cytoplasm. From these results, it was revealed that the accumulation of tau protein in the cytoplasm was observed in a part of the layer 5 nerve cells.

### «Immunostaining of tau protein and synaptophysin»

Subsequently, immunostaining of tau protein and synaptophysin in the cerebral organoid on the 84th day of culture, which had been prepared from human iPS cell line PS2-2 derived from an Alzheimer's disease patient, was carried out.

Fig. 15A, Fig. 15B and Fig. 15C are fluorescence photomicrographs showing the results of immunostaining. In Fig. 15A, "inner" indicates the central part of the organoid, and "outer" indicates the outer edge part of the organoid. The scale bar is 100 µm. In addition, Fig. 15B is an enlarged staining image of the outer edge part of the organoid of Fig. 15A. The scale bar is 10 µm. In addition, Fig. 15C is an enlarged staining image of the central part of the organoid of Fig. 15A. The scale bar is 10 µm.

As a result of the above immunostaining, synaptophysin was detected at the outer edge and the central part of the cerebral organoid. In a case where the photomicrograph was enlarged, a large number of puncta were observed along the neurites at the outer edge part; however, a staining image in which synaptophysin seemed to be aggregated was obtained at the central part. It is conceived that tau protein accumulates in the cytoplasm of the nerve cell in the central part of the cerebral organoid, abnormality in the formation of neurites occurs, and thus the synaptophysin, having lost its target destination, is aggregated.

### <<Immunostaining of tau protein and gammaH2A.X>>

Subsequently, immunostaining of tau protein and gammaH2A.X, which is an aging marker in the cerebral organoid, on the 84th day of culture, the cerebral organoid having been prepared from the human iPS cell line PS2-2 derived from an Alzheimer's disease patient, was carried out.

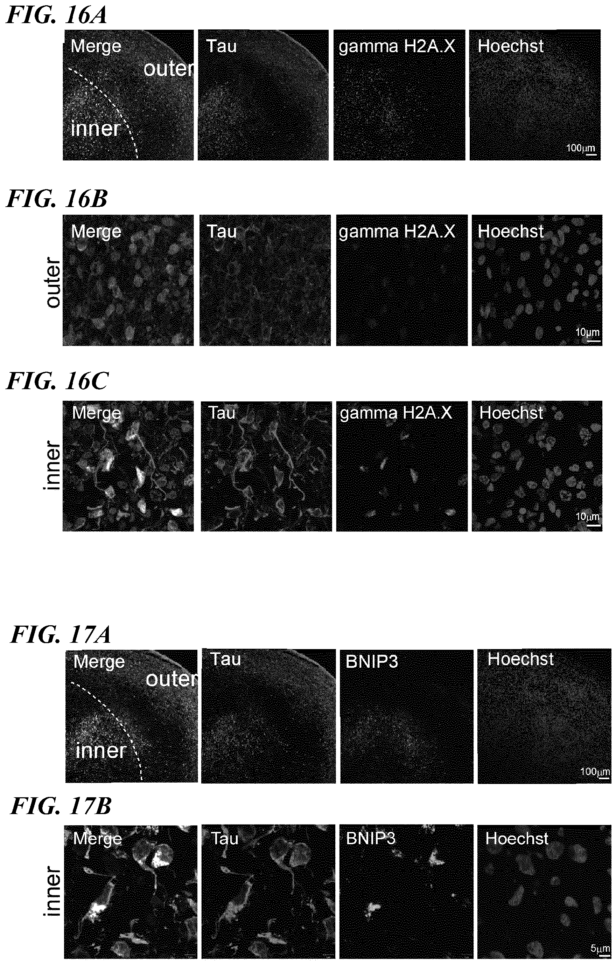

Fig. 16A, Fig. 16B and Fig. 16C are fluorescence photomicrographs showing the results of immunostaining. In Fig. 16A, "inner" indicates the central part of the organoid, and "outer" indicates the outer edge part of the organoid. The scale bar is 100 µm. In addition, Fig. 16B is an enlarged staining image of the outer edge part of the organoid of Fig. 16A. The scale bar is 10 µm. In addition, Fig. 16C is an enlarged staining image of the central part of the organoid of Fig. 16A. The scale bar is 10 µm.

As a result of the above immunostaining, gammaH2A.X was detected in the central part of the cerebral organoid. In a case where the photomicrograph was enlarged, there was almost no gammaH2A.X in the outer edge, but in the central part, gammaH2A.X was detected in the nerve cells in which tau protein accumulated. This result suggests that a phenomenon of cell senescence may occur in the cells in the central part of the cerebral organoid.

### «Immunostaining of tau protein and BNIP3»

Subsequently, immunostaining of tau protein and BNIP3, which is a hypoxic marker in the cerebral organoid, on the 84th day of culture, the cerebral organoid having been prepared from the human iPS cell line PS2-2 derived from an Alzheimer's disease patient, was carried out.

Fig. 17A and Fig. 17B are fluorescence photomicrographs showing the results of immunostaining. In Fig. 17A, "inner" indicates the central part of the organoid, and "outer" indicates the outer edge of the organoid. The scale bar is 100 µm. In addition, Fig. 16B is an enlarged staining image of the central part of the organoid of Fig. 16A. The scale bar is 5 µm.

As a result of the above immunostaining, BNIP3 was detected in the central part of the cerebral organoid, which suggested that the central part thereof is in a hypoxic state. In a case where the photomicrograph was enlarged, the expression of BNIP3 was seen in some of the cells in which tau protein accumulated. It has been reported that BNIP3 is present in the outer mitochondrial outer membrane and is involved in mitochondrial autophagy (mitophagy). For this reason, it was suggested that accumulation of mitochondria or abnormality in mitochondria may occur in these cells.

### [Experimental Example 7]

### (Detection of phosphorylated tau protein)

Phosphorylated tau protein in the cerebral organoid prepared in Experimental Example 1 was detected. On the 120th day of culture (day 120, 106 days after the start of stirring culture), each cerebral organoid was removed and fixed with 4% paraformaldehyde. Subsequently, the fixed organoids were embedded in an O.C.T. compound (KENIS, Ltd.) to prepare a frozen section.

Subsequently, each frozen section was immunostained with an anti-phosphorylated tau antibody (clone AT8, Thermo Fisher Scientific, Inc.) to detect the phosphorylated tau protein. In addition, immunostaining of HuC/D, which is a marker for the nerve cell, and GFAP, which is a marker for the astrocyte, was also carried out. In addition, nuclei were also stained with Hoechst33342.

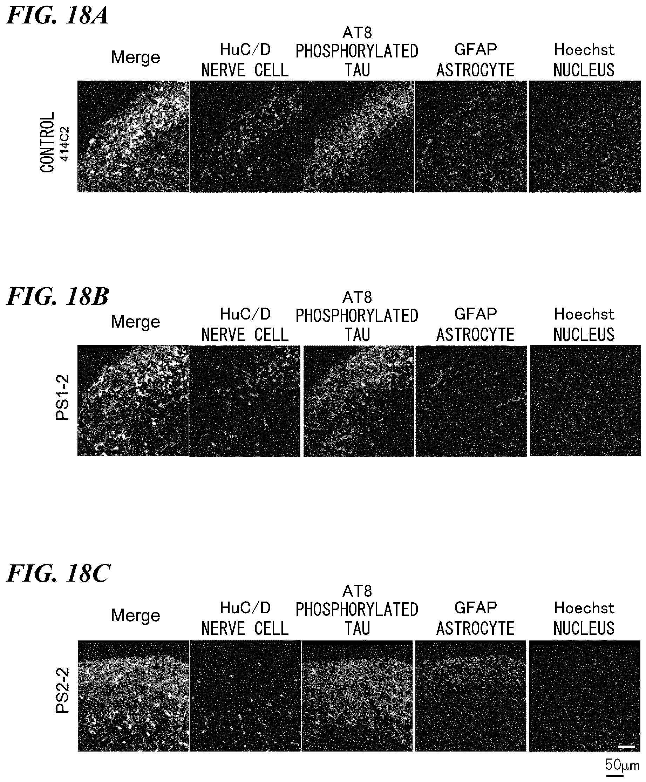

Fig. 18A, Fig. 18B amd Fig. 18C are fluorescence photomicrographs showing the results of immunostaining. Fig. 18A is a representative photographic image showing the result of the cerebral organoid on the 120th day of culture, which was prepared from the wild-type human iPS cell line 414C2. In addition, Fig. 18B is a representative photographic image showing the result of the cerebral organoid on the 120th day of culture, which was prepared from the human iPS cell line PS1-2 derived from an Alzheimer's disease patient. In addition, Fig. 18C is a representative photographic image showing the result of the cerebral organoid on the 120th day of culture, which was prepared from the human iPS cell line PS2-2 derived from an Alzheimer's disease patient. The scale bar is 50 µm. As a result of the above immunostaining, the presence of phosphorylated tau protein was observed in each cerebral organoid.

### [Experimental Example 8]

### (Examination of presence of tau protein having changed three-dimensional structure)

In the cerebral organoid prepared in Experimental Example 1, the tau protein having a changed three-dimensional structure was detected. On the 120th day of culture (day 120, 106 days after the start of stirring culture), each cerebral organoid was removed and fixed with 4% paraformaldehyde. Subsequently, the fixed organoids were embedded in an O.C.T. compound (KENIS, Ltd.) to prepare a frozen section.

Subsequently, each frozen section was immunostained with an MC1 antibody (provided by Dr. Peter Davies) known to recognize the tau protein having a changed three-dimensional structure. In addition, immunostaining of HuC/D, which is a marker for the nerve cell, and GFAP, which is a marker for the astrocyte, was also carried out. In addition, nuclei were also stained with Hoechst33342.

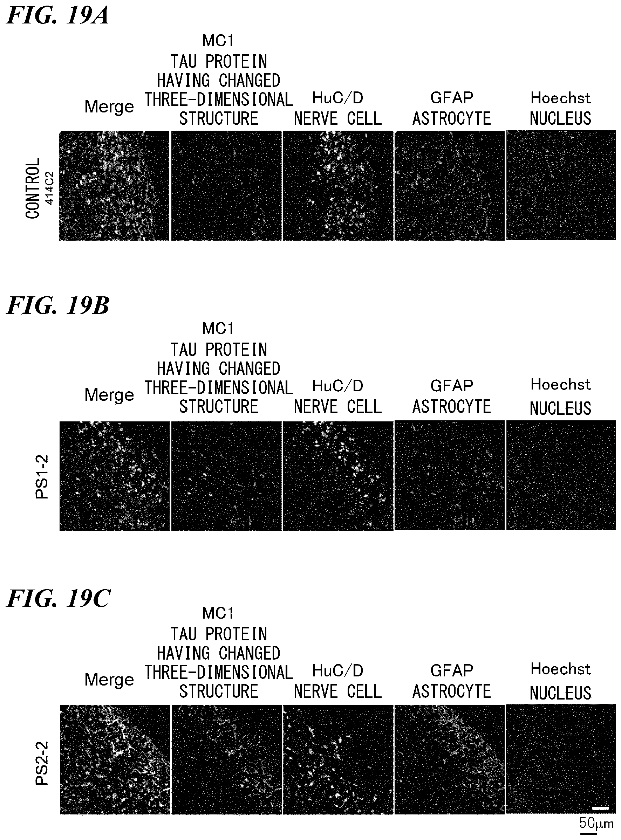

Fig. 19A to 19C are fluorescence photomicrographs showing the results of immunostaining. Fig. 19A is a representative photographic image showing the result of the cerebral organoid on the 120th day of culture, which had been prepared as a control from the wild-type human iPS cell line 414C2. In addition, Fig. 19B is a representative photographic image showing the result of the cerebral organoid on the 120th day of culture, which was prepared from the human iPS cell line PS 1-2 derived from an Alzheimer's disease patient. In addition, Fig. 19C is a representative photographic image showing the result of the cerebral organoid on the 120th day of culture, which was prepared from the human iPS cell line PS2-2 derived from an Alzheimer's disease patient. The scale bar is 50 µm.

As a result of the above immunostaining, a large number of MC1-positive cells indicating the presence of the tau protein having a changed three-dimensional structure were observed in astrocytes in the cerebral organoids derived from PS 1-2 and PS2-2, which are human iPS cell lines derived from Alzheimer's disease patients, as compared with the control cerebral organoid derived from 414C2.

### Industrial Applicability

According to the present invention, it is possible to provide a technique for efficiently forming a cerebral organoid having amyloid plaques.

## Claims

1. A production method for a cerebral organoid having amyloid plaques, the method comprising:
a step (a) of forming, in the presence of a SMAD inhibitor, an embryoid body from a pluripotent stem cell having a mutation in an Alzheimer's disease-related gene;
a step (b) of embedding the embryoid body after the step (a) in an extracellular matrix and three-dimensionally culturing the embedded embryoid body in the presence of a SMAD inhibitor and a glycogen synthase kinase 3β (GSK3β) inhibitor to form an organoid; and
a step (c) of removing the organoid after the step (b) from the extracellular matrix and subjecting the removed organoid to stirring culture in a medium,
wherein at least a part of the step (c) is carried out in the presence of leukemia inhibitory factor (LIF).

2. The production method according to Claim 1,
wherein at least a part of the step (c) is carried out in the presence of more than 20% by volume of oxygen.

3. The production method according to Claim 1 or 2,
wherein the step (c) is carried out for 100 days or more.

4. The production method according to any one of Claims 1 to 3, further comprising a step of culturing, before the step (a), the pluripotent stem cell in the presence of less than 100 ng/mL of fibroblast growth factor-2 (FGF2).

5. The production method according to any one of Claims 1 to 4,
wherein the pluripotent stem cell is cultured in a feeder-free manner.

6. The production method according to any one of Claims 1 to 5,
wherein the Alzheimer's disease-related gene is a presenilin 1 (PS1) gene, a presenilin 2 (PS2) gene, or a β-amyloid precursor protein (APP) gene.

7. A cerebral organoid having amyloid plaques of which the diameters are more than 20 µm.

8. The cerebral organoid according to Claim 7,
wherein in a cross section of the cerebral organoid, an average of a proportion of an area per amyloid plaque with respect to a total area of the cross section is 0.1% or more.

9. The cerebral organoid according to Claim 7 or 8,
wherein the number of the amyloid plaques is 2 or more per cerebral organoid.

10. The cerebral organoid according to any one of Claims 7 to 9,
wherein a molar ratio of an expression amount of amyloid β42 to an expression amount of amyloid β40 (the expression amount of amyloid p42/the expression amount of amyloid β40) is 0.15 or more.

11. The cerebral organoid according to any one of Claims 7 to 10,
wherein the cerebral organoid is produced by the production method according to any one of Claims 1 to 6.

12. A screening method for a therapeutic drug for Alzheimer's disease, the screening method comprising:
a step of culturing the cerebral organoid according to any one of Claims 7 to 10 in the presence of a test substance; and
a step of measuring the sizes of amyloid plaques of the cerebral organoid,
wherein a reduction of the sizes of the amyloid plaques indicates that the test substance is a therapeutic drug for Alzheimer's disease.

13. A screening method for a prophylactic drug for Alzheimer's disease, the method comprising:
a step (a) of forming, in the presence of a SMAD inhibitor, an embryoid body from a pluripotent stem cell having a mutation in an Alzheimer's disease-related gene;
a step (b) of embedding the embryoid body after the step (a) in an extracellular matrix and three-dimensionally culturing the embedded embryoid body in the presence of a SMAD inhibitor and a GSK3β inhibitor to form an organoid;
a step (c) of removing the organoid after the step (b) from the extracellular matrix and subjecting the removed organoid to stirring culture in a medium to obtain a cerebral organoid, at least a part of the step (c) being carried out in the presence of a test substance; and
a step (d) of measuring the sizes of amyloid plaques of the cerebral organoid, after carrying out the step (c) for 100 days or more,
wherein in the step (d), a reduction of the sizes of the amyloid plaques as compared with a control indicates that the test substance is a prophylactic drug for Alzheimer's disease.

14. A screening method for a prophylactic drug or a therapeutic drug for Alzheimer's disease, the method comprising:
a step (a) of forming, in the presence of a SMAD inhibitor, an embryoid body from a pluripotent stem cell having a mutation in an Alzheimer's disease-related gene;
a step (b) of embedding the embryoid body after the step (a) in an extracellular matrix and three-dimensionally culturing the embedded embryoid body in the presence of a SMAD inhibitor and a GSK3J3 inhibitor to form an organoid;
a step (c) of removing the organoid after the step (b) from the extracellular matrix and subjecting the removed organoid to stirring culture in a medium to obtain a cerebral organoid, at least a part of the step (c) being carried out in the presence of a test substance; and
a step (d') of quantifying expression amounts of amyloid β40 and amyloid β42 expressed by the cerebral organoid of the step (c),
wherein in the step (d'), a reduction of a ratio of an expression amount of amyloid β42 to an expression amount of amyloid β40 as compared with a control indicates that the test substance is a prophylactic drug or therapeutic drug for Alzheimer's disease.


### Sequence Listing

SEQUENCE LISTING

<110>  Keio University

<120>  Method for producing cerebral organoid

<130>  PC-29991

<150>  JP2019-126266
<151>  2019-07-05

<160>  2

<170>  PatentIn version 3.5

<210>  1
<211>  40
<212>  PRT
<213>  Homo sapiens

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  2
<211>  42
<212>  PRT
<213>  Homo sapiens

<400>  2